# EUROPEAN PATENT SPECIFICATION

(11) **EP 1 503 732 B1**
(45) Date of publication and mention of the grant of the patent: **08.10.2008**
(21) Application number: 03747606.6
(22) Date of filing: 28.04.2003
(51) Int. Cl.: A61K 9/00, A61K 47/34, A61K 31/765

(54) **REDUCED FORMATE POLY(ALKYLENE OXIDES) WITH REBOXETINE FOR REDUCING IMPURITY FORMATION**
POLY(ALKYLENOXIDE) VON REDUZIERTEM FORMAT MIT REBOXETIN ZUR VERRINGERUNG DER BILDUNG VON VERUNREINIGUNGEN
POLY(ALKYLENE OXYDES) DE FORMATE REDUITS AVEC LA REBOXETINE PERMETTANT LA REDUCTION DE LA FORMATION D'IMPURETES

(30) Priority: 29.04.2002 US 376481 P
(43) Date of publication of application: 09.02.2005
(73) Proprietor: ALZA Corporation, Mountain View, CA 94039-7210 (US)
(72) Inventor: SEROFF, Sylvia, L., Sunnyvale, CA 94086 (US); YAM, Noymi, V., Sunnyvale, CA 94086 (US); AYER, Atul, D., Palo Alto, CA 94303 (US); LAM, Andrew, C., South San Francisco, CA 94080 (US)
(74) Representative: Mercer, Christopher Paul
(86) International application number: PCT/US2003/013156
(87) International publication number: WO 2003/092649

(56) References cited:
- WO-A-01/19337
- WO-A-02/088217

## Description

### FIELD OF THE INVENTION

The present invention relates to the use of poly(alkylene oxide) polymers having reduced amounts of formic compounds with reboxetine to reduce formation of impurities in osmotic controlled release pharmaceutical dosage forms incorporating reboxetine.

### BACKGROUND OF THE INVENTION

Pharmaceutical drug delivery vehicles commonly utilize poly(alkylene oxide) polymers for controlled release dosage forms delivering therapeutic agents. Dosage forms incorporating therapeutic agents such as secondary amines including reboxetine, however, are unable to incorporate traditional poly(alkylene oxide) polymers because the high formate content reacts with the secondary amine creating an unstable dosage form. As such, controlled release dosage forms delivering secondary amines like reboxetine instead previously used a carbohydrate such as maltodextrin as the carrier to provide a stable dosage form and longer shelf life. See U.S. Patent Application No. 09/661,976 assigned to ALZA Corporation.

Application No. 09/661,976 discloses the use of a carbohydrate in combination with the reboxetine because the carbohydrate exhibits minimal reactivity with the reboxetine molecule, which contains a relatively reactive secondary amine moiety that may react with excipients and impurities forming degradation products. However, while this combination suffices for the drug composition in the dosage form, the prior art taught the use of a barrier layer between the drug layer and the traditional poly(alkylene oxide) polymer of the expandable push layer to eliminate any contact between the secondary amine drug and the poly(alkylene oxide) polymer necessary in the push layer.

Secondary amine reboxetine methanesulfonate is a pharmaceutically acceptable salt form of the drug, which has been available outside of the United States in an immediate-release oral dosage form product of Pharmacia and Upjohn Co. and is currently being evaluated for marketing in the United States.

US Patent No. 5,804,209 also describes pharmaceutical compositions containing bioadhesive starches and drugs, including, *inter alia*, reboxetine, primarily for the delivery of drug by the nasal route, although other routes of administration are mentioned. The bioadhesive nature of the starches is described as increasing the time at which the drug remains at the absorption site as compared to drug released from non-bioadhesive compostions. US Patent Nos. 6,028,070; 6,046,193; and 6,066,643 describe, respectively, a method that uses reboxetine to treat oppositional defiant disorder, a method that uses reboxetine to treat attention-deficit disorder, and pharmaceutical compositions and methods using reboxetine in combination with moxonidine.

Poly(alkylene oxide) polymers are sold in a wide range of molecular weights ranging, for example, from about 100,000 g/gmole to 10,000,000 g/gmole. For certain applications, e.g., pharmaceutical applications, poly(alkylene oxide) polymers in a lower molecular weight range, e.g., from about 100,000 to 2,000,000 g/gmole, are often desired. Commercial production processes for the manufacture of poly(alkylene oxide) polymers produce products typically having a molecular weight of about 4,000,000 g/gmole or higher. Typically, these higher molecular weight poly(alkylene oxide) polymer products are irradiated in the presence of oxygen with a suitable irradiation source, such as, for example, gamma rays from a ⁶⁰Co source or an electron beam from a Vandegraaff Generator. The details of such irradiation processes are known to those skilled in the art. As a result of the irradiation, various oxidation by-products are produced, such as, for example, hemiformal groups (ROCH₂OH) and formate groups (ROCHO) attached to the ends of polymer chains ("R"), as well as various volatile oxidation products, such as, for example, formaldehyde, carbon dioxide, carbon monoxide, ethylene glycol, glycol aldehyde and mono and diformates of ethylene glycol. See, for example, Radiation Induced Oxidation Of Solid Polyethylene Oxide, Christian Decker, Journal of Polymer Science, Vol. 15, 781-798 and Vol. 15, 799-813, John Wiley & Sons, Inc (1977).

WO0119337 describes dosage forms and methods for providing sustained release of reboxetine. The sustained release dosage forms provide therapeutically effective average steady-state plasma reboxetine concentrations when administered once per day. This once-a-day dosing regimen results in only one peak plasma reboxetine concentration occurrence in each 24 hour period. In addition, the peak plasma reboxetine concentration occurs at a later time following dose administration and exhibits a lesser magnitude than the peak plasma reboxetine concentration that occurs following administration of reboxetine in an immediate-release dosage form.

In view of the above, it would be an advance in the art to provide methods and compositions for providing patients with effective steady-state plasma reboxetine concentrations while providing reduced impurity formation, Reduced impurities provide longer periods of stability for the compositions and dosage forms allowing preparation of dosage forms that exhibit longer shelf life, adding to the economic benefits of the controlled release dosage form. Use of the poly(alkylene oxide) polymer would provide a preferred composition over carbohydrate if the poly(alkylene oxide) polymer had lower reactivity with reboxetine. In addition, it would be an advance to provide methods and compositions for providing patients with effective steady-state plasma reboxetine concentrations through an controlled release dosage form using the poly(alkylene oxide) polymer while providing an extended shelf life. It would further be an advance in the art to provide a dosage form for delivering secondary amine drugs without the use of a barrier layer between the drug containing composition and the push layer containing poly(alkylene oxide) polymer.

In accordance with the present invention, it has been found that the oxidation by-products which are comprised of formic compounds, e.g., formic acid and esters and salts thereof, can Impart undesirable characteristics to the poly(alkylene oxide) polymers in particular for pharmaceutical applications. It would be desirable to provide poly(alkylene oxide) polymers having reduced amounts of oxidation by-products resulting from irradiation of the polymers, particularly formic compounds.

### SUMMARY OF THE INVENTION

In accordance with the present invention, poly(alkylene oxide) polymers having reduced i.e. less than 200 ppmw amounts of formic compounds, e.g., formic acid and esters and salts thereof, are provided. Quite surprisingly in accordance with the present invention, it has been found that by removing the formic compounds which are free, i.e., not chemically bonded to the poly(alkylene oxide) chain, can significantly improve the characteristics of the poly(alkylene oxide) polymers.

It is now possible to treat poly(alkylene oxide) polymers which have been subjected to irradiation in order to reduce the amount of formic compounds. The poly(alkylene oxide) polymers are contacted with a treatment acid having Pka lower than that of formic acid to replace the formic compounds with the treatment acid.

In another aspect, the invention comprises a sustained release composition comprising reboxetine or its pharmaceutically acceptable salts and the reduced formate poly(alkylene oxide) polymer.

In another aspect, the invention comprises a sustained release dosage form adapted to release reboxetine, or a pharmaceutically acceptable acid addition salt thereof and the poly(alkylene oxide) polymer over an extended period of time.

In another aspect, the invention comprises sustained release dosage form comprising a reboxetine composition and poly(alkylene oxide) polymer without the need for a barrier layer between the drug composition layer and the expandable push layer of the osmotic delivery system. More particularly, the invention describes a composition comprising the reboxetine and the poly(alkylene oxide) polymer requiring no coating, encapsulation or other barrier between the two components while still having minimal degradation.

The above-described features and advantages as well as others will become more apparent from the following detailed disclosure of the invention and the accompanying claims.

### DETAILED DESCRIPTION OF THE INVENTION

The poly(alkylene oxide) polymers suitable for use in accordance with the present invention comprise polymers of alkylene oxides containing from 1 to about 4 carbon atoms per molecule, e.g., ethylene oxide or propylene oxide, as well as copolymers and derivatives thereof.

Preferably, the poly(alkylene oxide) polymers of the present invention are ethylene oxide polymers. The ethylene oxide polymers include, for example, homopolymers of ethylene oxide and copolymers of ethylene oxide with one or more polymerizable comonomers, usually olefin oxide comonomers. The particular comonomer, when used in accordance with the present invention, is not critical and may contain hydrocarbon substituents such as alkyl, cycloalkyl, aromatic, alkene and branched alkyl groups. However, the amount of comonomer, e.g., 1,2-propylene oxide, must not exceed that which would cause the poly(ethylene oxide) to become insoluble in water. Typical olefin oxide comonomers include 1,2-propylene oxide, 2,3-butylene oxide, 1,2-butylene oxide, styrene oxide, 2,3-epoxy hexane, 1,2-epoxy octane, butadiene monoxide, cyclohexene monoxide, epichlorohydrin, and the like.

The secondary amine drug, reboxetine methanesulfonate was found to be unstable in the presence of traditional polyethylene oxides. Substantial levels of various impurities are formed, which increase in concentration over time. The predominant impurity that is formed is the n-formyl species of reboxetine methanesulfonate, known as n-formyl reboxetine.

In addition, the secondary amine drug, reboxetine methanesulfonate is substantially less stable in the presence of traditional poly(alkylene oxide) polymer that has been subjected to irradiation processes or oxidation. Poly(alkylene oxide) polymer consists of long chains of the polymer. Irradiation and oxidation cause these chains to be cleaved, leaving inorganic and organic formates at the ends of the chains. As more cleaving occurs, there are higher levels of formates present. It appears that when the secondary amine reboxetine methanesulfonate is placed in close contact with the formate compounds of the traditional poly(alkylene oxide) polymer, the degradation increases and the levels of all impurities including n-formyl reboxetine are drastically increased.

The present invention utilizes a reduced formate polymer manufactured through a process that removes formates from the traditional poly(alkylene oxide) polymer by treatment with a reagent such as strong acid to produce a reduced formate poly(alkylene oxide) polymer. The process for reducing the amount of formic compounds from the polymer and the reduce formate polymers are described in PCT International Publication Number WO 02/088217A1 assigned to Union Carbide Chemicals & Plastics Technology Corporation. This approach also lowers final pH of the reduced formate poly(alkylene oxide) polymer. The concentration of formates and the pH level of the poly(alkylene oxide) polymer are related to the reactivity of the poly(alkylene oxide) polymer when in contact with secondary amines. The present invention further surprisingly provides a stable composition of a poly(alkylene oxide) polymer and a secondary amine.

The Dow Chemical Company of Midland, MI manufactures the reduced formate polymers included in the present invention. Further aspects of the process for reducing the formate content in the poly(alkylene oxide) polymer are discussed herein including Examples 1 - 8.

The reagent can be any strong acid capable of reducing the formates and thus, the impurity formation. One such strong acid is phosphoric acid. Another such strong acid, and a preferred acid, is methanesulfonic acid based on the nature of the drug, being a methanesulfonate. The use of an acid containing the same functional group that is present in the drug substance appears to further reduce the possibility of impurity formation related to the poly(alkylene oxide) polymer washing technique such as phosphate formation when using phosphoric acid.

Commercial processes for manufacturing poly(alkylene oxide) polymers typically employ a catalyst, or combination of catalysts, which is consumed in the polymerization. One catalyst comprises a reaction product of calcium and ammonia. Catalysts of this type are further described in U.S. Patent Nos. 4,193,892 and 4,267,309 issued to Goeke, et al., and U.S. Patent No. 4,667,013 issued to Reichle. Another catalyst comprises an organo zinc compound such as, for example, diethyl zinc, e.g., diethyl zinc and water or an alcohol. Catalysts of this type are further described for example, in U.S. Patent No. 5,326,852, issued to Masato et al. and Japanese Patent Nos. JP 46007709, JP 45007751 and JP 5302731. Still another catalyst is an aluminum-based catalyst, known as Vandenberg catalysts. Catalysts of this type are further described for example, in U.S. Patent Nos. 3,135,705, 3,219,591 and 3,403,114 issued to E.J. Vandenberg et al. Further details concerning suitable catalysts are known to those skilled in the art.

Further details concerning the preparation of poly(alkylene oxide) polymers of the present invention are known to those skilled in the art and disclosed in the literature; see, for example, U.S. Patent Nos. 2,969,403, issued to Helmut, et al.; 3,037,943, issued to Bailey, et al.; 3,167,519, issued to Bailey, et al.; 4,193,892, issued to Geoke, et al.; and 4,267,309, issued to Geoke, et al. Moreover, the polymers can be prepared using conventional apparatus known to those skilled in the art.

Typically, the poly(alkylene oxide) polymers which have been irradiated to reduce the molecular weight have a weight average molecular weight greater than about 100,000 to 2,000,000, more typically from about 100,000 to 1,000,000 and often from about 300,000 to 1,000,000 grams per gram mole ("g/gmole"). Techniques for determining the weight average molecular weight of poly(alkylene oxide) polymers are known to those skilled in the art. One such technique is gel permeation chromatography. As noted above, irradiation processes suitable to reduce the molecular weight of poly(alkylene oxide) polymers are known to those skilled in the art, and such processes are not critical to the present invention.

Preferably, the poly(alkylene oxide) polymers of the present invention are water-soluble. As used herein, the term "water-soluble" means that at least 0.1 gram and preferably at least 1 gram of the poly(alkylene oxide) are soluble in 100 grams of distilled water at 25° C at one atmosphere. Techniques for varying the water solubility of poly(alkylene oxide) polymers are known to those skilled in the art.

The particular physical form of the poly(alkylene oxide) polymers is not critical to the present invention. Typically, the physical form selected from tablets, pellets, powder, granules or extrudates.

In powder form, the particle size of the poly(alkylene oxide) polymers is typically from less than about 40 to 2000 microns, preferably with less than about 65 percent of the particles having a particle size greater than about 150 microns. A convenient way to measure the particle size of the polymer is to use sieves corresponding to standard mesh sizes. Sieves having the mesh values corresponding to the U.S. Standard Sieve and Tyler Equivalents, Table 21-12, CHEMICAL ENGINEERS HANDBOOK, Perry and Chilton, 5th Edition, McGraw-Hill Book Company are suitable for use herein.

When in the form of granules, i.e., a large particle comprising a composite of numerous smaller particles, tablets or pellets, the particle size is typically from about 0.1 to 5 millimeters. When in the form of extrudates, the poly(alkylene oxide) polymer particles have an average diameter of from about 2 to 10 millimeters. When in granular, tablet, pellet or extrudate form, the particles may comprise other polymers or materials in addition to the poly(alkylene oxide) polymer.

Further details concerning the processes and equipment suitable for manufacturing the poly(alkylene oxide) polymer particles of the present invention, e.g., in the form of powder, pellets, granules, tablets or extrudates, are known to those skilled in the art.

As used herein, the term "formic compound" means any compound or group within a compound or polymer which has a formic acid group or salt or ester thereof. Typically, the amount of the formic compound in the poly(alkylene oxide) starting materials of the present invention is from about 200 to 2,000 ppmw, more typically from about 300 to 1,500 ppmw, based on the total weight of the polymer. Preferably, the formic compounds which are treated are those which are free, i.e., not chemically bonded to the poly(alkylene oxide) chain. Examples of such free formic compounds include formic acid, calcium formate, sodium formate, methyl formate, isopropyl formate and ethylene glycol formate. For poly(ethylene oxide) polymers produced in basic pH, such as POLYOX® produced by The Dow Chemical Company of Midland, MI, the free formic compounds are also referred to herein as "inorganic formates".

The poly(alkylene oxide) polymer particles comprising the formic compound can be treated in order to reduce the amount of the formic compound, particularly on or near the surface of the polymer particle, by contacting the surface of the particle with an acid, i.e., a treatment acid, having a Pka lower than the Pka of formic acid, i.e., 3.75. Thus, the treatment acid can release the formic acid from the formic compound in the polymer particle. The Pka is a measurement of the completeness of an incomplete chemical reaction. It is defined as the negative logarithm (to the base 10) of an equilibrium constant ("K") for the reaction in question. Further details concerning the Pka of weak acids and the measurement thereof, are known to those skilled in the art. Preferred treatment acids are selected from the group consisting of hydrofluoric acid (Pka = 3.14), sulfuric acid (Pka = 0.4), nitric acid, thiosurfuric acid (Pka = 2.00), phosphoric acid (Pka = 2.12), citraconic acid (Pka = 2.48), citric acid (Pka = 3.06), dichloroacetic acid (Pka = 1.30), oxalic, acid (Pka = 1.19), methanesulfonic acid (Pka < 3.75), malonic acid (Pka = 2.85) and mixtures thereof.

The particular method of treating the poly(alkylene oxide) polymer with the treatment acid is not critical to the present invention.

The process may comprise (i) forming a slurry of the polymer particle in a liquid medium comprising the treatment add under conditions effective to promote the extraction of the formic compound from the polymer particle; and (ii) recovering a treated polymer particle having a reduced amount of the formic compound. In this process the liquid medium is preferably a non-solvent for the poly(alkylene oxide) polymer. Preferred non-solvents include any inert organic solvents which are at least partially miscible with water. Examples of useful organic solvents include alcohols, ketones, glycol ethers, hydroxyl esters, alkyl pyrolidones, toluene, isopentane, tetrahydrofuran, dioxanes, chlorinated solvents, and ethers. The preferred solvents include acetone, methyl ethyl ketone, and isopropyl alcohol. The amount of liquid medium used in comparison to the poly(alkylene oxide) is not critical. However, it is convenient to use a liquid medium to polymer ratio from about 100 to about 2000 milliliters ("ml") of liquid medium per 100 gram of poly(alkylene oxide), preferably from 200 to 1000 ml per 100 gram of poly(alkylene oxide).

The acid concentration has a direct effect on the efficacy of the extraction process. Usually, the higher the acid concentration the more efficient the extraction process. Preferred acid concentrations range from about 1 to 1000 millimoles("mM") of acid per 100 gram poly(alkylene oxide) and more preferably from about 50 to 500 mM/100 gram poly(alkylene oxide). It is also preferred to include a sufficient amount of water to swell or partially solubilize the polymer to enhance the efficiency of the replacement of the formic compound with the treatment acid. The amount of water may typically vary from about 0.01 to 25 gram of water per 100 gram of poly(alkylene oxide) with the proviso that the amount is large enough to enhance the extraction process while not too large to cause unwanted agglomeration of the polymer particles. The preferred water charge ranges from about 0.1 to 5 gram per 100 gram of poly(alkylene oxide).

The extraction process is typically carried out at ambient temperature and pressure, e.g., 25°C and 1 atm. However, the preferred temperature is between 20 and 50°C such that it is below the melting point of the polymer. If the reaction is carried out at a temperature too close to, or over, the melting point of the polymer, agglomeration of the polymer particles may result. The extraction time is not critical and may typically vary from 1 minute up to 24 hours. The preferred extraction time is from about 30 minutes to about 3 hours. The extraction process is preferably carried out In either a stainless steel vessel, a glass-lined steel vessel, or a glass or ceramic vessel.

After the extraction step, the polymer is filtered and dried. Filtration of the polymer, after extraction, from the liquid medium may be accomplished by means known to those skilled in the art, e.g., by using either a filter or a centrifuge. The drying operation may be carried out, for example, using either a forced air oven or a vacuum oven. The temperature of such an oven must be carefully controlled to below the melting point of the polymer in order to minimize the risk of polymer agglomeration during drying. The referred drying operation is to dry the polymer under vacuum under a stream of nitrogen at a temperature about 15°C below the melting point of the polymer. The dried polymer should be free-flowing particles similar to its precussor. Certain additives such as antioxidant, pigment, dye, lubricant, flowing aid, or filler may be added to the dried product if so desired.

While any suitable equipment for washing, filtration, centrifugation, vacuum drying, drying, and mixing may be used for the purpose of conducting the extraction process of this invention, a Nutsche Filter(or its equivalent, such as, for example, a Zwag Filter or a Rosenmund Filter) may be particularly useful. When a Nutsche Filter is used, the extraction and filtration process may be conducted in the same apparatus. As a result, the need for material transferring and the need for equipment cleaning are minimized. Additionally, because the operations are carried out In a single apparatus, contamination by foreign materials is also minimized.

Alternatively, the process comprises (i) applying a sufficient amount of the treatment acid to the surface of the polymer particle to promote the replacement of the formic compound on the surface with the treatment acid; and (ii) removing the replaced formic compound from the particle.

In this process a sufficient quantity of the treatment acid is applied to the surface of the polymer particle, e.g., by spraying in order to promote the exchange of the treatment acid with the formic compound. Thereafter, the formic compound is removed from the polymer particle through evaporation, e.g., by heating in an oven as described above, e.g., at a temperature of at least about 15° C less than the melting point of the polymer.

Alternatively, the extraction may be conducted in a homogeneous system. In this aspect, the poly(alkylene oxide) is dissolved in a suitable solvent such as water and contacted with the treatment acid. The contacting of the polymer with the treatment acid can be accomplished by adding an aqueous solution of the treatment acid to the solution of the dissolved polymer. The solution is allowed to stand or is mixed for a short duration which can be determined by one skilled in the art. The polymer is recovered by coagulation in a nonsolvent such as acetone or isopropyl alcohol. The coagulated polymer is preferably rinsed with an additional amount of nonsolvent, collected by filtration, and dried. The homogeneous process described above is preferred when it is desired to reduce the formic compound to a lower level than the slurry process described above.

After removal of the formic compound, the average concentration of the formic compound in the particle is less than 200 ppmw, preferably from about 10 to 150 ppmw, more preferably below 120 ppmw and most preferably below 100 ppmw, based on the total weight of the particles. It is preferred that once treated polymer be stabilized by a suitable antioxidant or a mixture of antioxidants, the details of which are known to those skilled in the art. Examples of such antioxidants include butylated hydroxytoluene and Vitamin E.

In one aspect of the invention, the product produced is a particle (i) a polymer polymerized from an alkylene oxide monomer; and (ii) a formic compound; said particle having center portion and an outer surface and further having a concentration gradient of the formic compound wherein a higher concentration of the formic compound thereof is present in the center portion than at the outer surface. It is preferred that the concentration gradient has less than about 5 wt.% of the formic compound present in the outer most 10% of the particle along a radial cross-section of the particle beginning in the center of the particle and ending at its surface.

The product produced by the acid-treatment process of this invention may have either an acidic, basic, or neutral pH depending on the product treatment after the acid-washing step. For certain pharmaceutical applications, it is advantageous to leave a sufficient amount of acid in the product to give it an acidic pH.

The poly(alkylene oxide) polymers produced in accordance with the present invention have a variety of end uses. Typical end uses include, for example, thickener for latex paints, an excipient for the delivery of drugs, such as in the controlled-release of drugs in pharmaceutical applications, filler retention and drainage aid in paper manufacture, lubricant for safety razors, binder for ceramics, water-soluble seed tapes, concrete pumping aid and flocculant for the mining industry. In many of the above-mentioned uses, the poly(alkylene oxide) polymers are combined with other polymers or substances. Typical polymers and substances include, for example, other water-soluble polymers such as, for example, polyvinyl pyrrolidone, polyacrylamide, hydroxypropyl cellulose, polyvinyl imidazoline, and polyhydroxyethylmethacrylate; hair softeners; oils such as silicone oil and mineral oil; substances that enhance the healing or stop the bleeding of the skin; essential oils such as menthol, eugenol, eucalyptol, safrol, and methyl salicylate; rinsing aids; non-volatile cooling agents; inclusion complexes of skin-soothing agents with cyclodextrin; fragrances; vitamin E (including common forms of vitamin E such as vitamin E acetate); vitamin A and B-carotene; panthenol and aloe; drugs, therapeutic agents; antipruritic/counterirritant materials; antimicrobial/keratolytic materials; antiinflammatory agents; antioxidants such as butylated hydroxytoluene; lubricants such as magnesium stearate; sugars such as lactose; fillers such as titanium oxide and starch, and astringents. The amount of such other polymers and substances is dependent upon the particular product to be manufactured. Further details concerning the selection and amounts of other polymers and substances useful with the poly(alkylene oxide) polymers of the present invention can be determined by those skilled in the art.

The invention is hereafter described with respect to the examples.

### Example 1

A slurry containing 70 g of POLYOX® WSR N80 NF( a poly(ethylene oxide) having a molecular weight of 300,000 produced by Union Carbide of Danbury, CT), 20 ml of hydrochloric acid(37%), and 480 ml of isopropyl alcohol(IPA) was stirred at room temperature for 60 minutes. The polymer was collected by filtration using a vacuum filter. The filter cake was washed with 100 ml of isopropyl alcohol ("IPA") two times and followed by a final rinse with 100 ml of acetone. The washed polymer was then collected and dried at 30°C under vacuum. The finished polymer was a free-flowing solid. The inorganic formate in the polymer was analyzed with an Ion Chromatography ("IC") instrument and the value was found to be 150 ppmw. The unwashed poly(ethylene oxide) had an inorganic formate level over 500 ppmw.

### Example 2

Example 1 was repeated with the exception that the hydrochloric acid was replaced with 5g of nitric acid (69.7%) and 15g of distilled water was added. The IPA rinse was done 3 times and followed by a final acetone rinse. The vacuum drying was conducted at 40°C instead of 30°C. The finished polymer was a free-flowing solid. IC analysis showed the inorganic formate level was reduced from over 500 ppmw to 130 ppmw.

### Example 3

Example 2 was repeated with the exception that the nitric acid was replaced with 4g of phosphoric acid (85%) and 16g of distilled water was added. Vacuum drying was conducted at 30°C over a weekend. The finished polymer was a free-flowing solid. IC analysis showed an inorganic formate reduction of from over 500 ppmw to 170 ppmw.

### Example 4

A slurry containing 100g of the poly(ethylene oxide) used in Example 1, 21.75g of methanesulfonic acid (99.5%) and 19g of distilled water is stirred at room temperature for 2 hours. The polymer was collected by vacuum filtration, rinsed twice with 70 ml of IPA. The polymer was then dried at 40°C under vacuum over a weekend. The finished polymer was a free-flowing solid. IC analysis showed an inorganic formate level of 48 ppmw. The unwashed poly(ethylene oxide) had a inorganic formate level of over 500 ppmw.

### Example 5

Example 4 is repeated with the exception that the anhydrous methanesulfonic acid was replaced with 20.6g of an aqueous methanesulfonic acid (70%) and 8.2g of distilled water. The finished polymer was a free-flowing solid. IC analysis showed an inorganic formate content of 48 ppmw. The unwashed poly(ethylene oxide) had an inorganic formate level of over 500 ppmw.

### Example 6

Example 4 was repeated with the exception that no distilled water was added to the slurry. The finished polymer had an inorganic formate level of 96 ppmw. The unwashed poly(ethylene oxide) had an inorganic formate level of over 500 ppmw.

### Example 7

A slurry containing 52g of POLYOX WSR^{®} N80NF, 20ml of hydrofluoric acid (49%), and 480 ml of IPA was mixed in a polypropylene beaker under nitrogen for 30 minutes. The polymer was collected by filtration under vacuum, and re-slurried in a fresh solution having the same composition as described above in this example for another 30 minutes. The polymer was collected by filtration under vacuum, rinsed twice with 100 ml of IPA, and followed by another rinse with acetone. The polymer was then dried under vacuum at RT for overnight. The dried polymer was blended with 19 mg of BHT. A free-flowing polymer was obtained.

### Example 8

A series of experiments was carried out using POLYOX WSR^{®} N80NF and acidic solutions containing different ratios of methanesulfonic acid (MSA) and distilled water in IPA for different lengths of washing time. The level of inorganic formate in the POLYOX WSR^{®} N80NF starting material was 471 ppmw. The formulations, washing conditions, and the level of inorganic formate found in the finished polymers are complied in the following table:

| MSA,g (70%) | IPA, g | Water,g | Washing Time,hr | Inorganic Formate, ppm |
|---|---|---|---|---|
| 10.3 | 275.1 | 4.1 | 0.5 | 114 |
| 41.2 | 275.1 | 4.1 | 0.5 | 88 |
| 10.3 | 825.3 | 12.5 | 0.5 | 100 |
| 41.2 | 825.3 | 12.5 | 0.5 | 80 |
| 10.3 | 825.3 | 12.5 | 4 | 80 |
| 41.2 | 825/3 | 12.5 | 4 | 31 |
| 41.2 | 275.1 | 4.1 | 4 | 27 |
| 10.3 | 275.1 | 4.1 | 4 | 48 |
| 25.75 | 550.2 | 8.32 | 2.25 | 83 |

### Example 9

### Dry blends of reboxetine with untreated POLYOX^{®} N-80, untreated POLYOX^{®} N-303 and hydrofluoric acid treated POLYOX^{®} N-80

Dry blended mixtures of reboxetine methanesulfonate with POLYOX^{®} were prepared to test the physical and chemical stability of the drug in the presence of lots of this polymer that contain different levels of inorganic formates. The blends were made by mixing 20 mg of reboxetine methanesulfonate with 80 mg of POLYOX^{®} in glass scintillation vials. The open vials were stored under controlled conditions at 40°C and 75% relative humidity (RH). The stability of the drug mixture was determined by detecting the level of the N-formyl species of reboxetine, which is known to be a dominant degradation product, that is present in the mix. The following table summarizes the stability of reboxetine with the lots of POLYOX^{®} that were tested.

| Material | Inorganic formate level (ppm) | Amount of N-formyl at 2 months stored at 40°C/75%RH (Weight % of drug) |
|---|---|---|
| POLYOX^{®} 200K (irradiated) | 473 | 0.76% |
| POLYOX^{®} 7000K (non-irradiated) | 90 | 0.02% |
| POLYOX^{®} 200K (irradiated) washed with hydrofluoric acid | 121 | 0.13% |

### Example 10

### 4 mg, 5/16" standard round system made with phosphoric-acid treated POLYOX^{®} N-80 with inorganic formate level of 37 ppm

POLYOX^{®} WSR N80 NF was treated with phosphoric acid to reduce the inorganic formate level to 37 PPM. This material was used in the drug layer of an osmotic reboxetine delivery system that was manufactured as follows:

First, a binder solution was prepared. 300 g of poly(vinylpyrrolidone) identified as K29-32 having an average molecular weight of 40,000 was dissolved in 2700 g of water. Then, 504.0 g of reboxetine methansulfonate, 8368 g of the phosphoric acid treated POLYOX^{®} WSR N80 NF, and 800.6 g of sodium chloride were added to a Freund Fluid Bed Granulator's bowl. The bowl was attached to the granulator and the granulation process was initiated for effecting granulation. Next, the dry powders were air suspended and mixed. Then, the binder solution was sprayed from a nozzle onto the powder. The granulating conditions were monitored during the process as follows: total solution spray rate of 120 g/min and inlet temperature of 40° C.

While spraying the binder solution, the filter bags were shaken for 10 seconds after every 30 second spray cycle to unglue any possible powder deposits. At the end of the solution spraying, 3000 g, the coated granulated particles were continued with the drying process. The machine was turned off, and the coated granules were removed from the granulator. The coated granules were passed through a 7 mesh screen using a Granumill. Next, the dried and screened granulation was transferred to a Gemco blender, mixed and lubricated 1.87 g of butylated hydroxytoluene for 10 minutes. Finally, 46.8 g of magnesium stearate was mixed into the granulation for 1 minute.

Next, a push composition was prepared as follows: first, a binder solution is prepared. 3.913 kg of hydroxpropymethylcellulose comprising a 11,200 molecular weight was dissolved in 45.0 kg of water.

Next, 36,000 g of sodium chloride and 600 g of ferric oxide was sized using a Quadro Comil with a 21-mesh screen. Then, all the screened materials, 77,040 g of pharmaceutically acceptable poly(ethylene oxide) comprising a 7,000,000 molecular weight, and 3,000 g of hydroxpropymethylcellulose comprising a 11,200 molecular weight are added to a Glatt Fluid Bed Granulator's bowl. The bowl was attached to the granulator and the granulation process was initiated for effecting granulation. Next, the dry powders were air suspended and mixed. Then, the binder solution was sprayed from 3 nozzles onto the powder. The granulating conditions were monitored during the process as follows: total solution spray rate of 700 g/min; inlet temperature 45° C; and process airflow of approximately 3000 m³/hr.

While spraying the binder solution, the filter bags were shaken for 15 seconds every 1.5 minutes to unglue any possible powder deposits. At the end of the solution spraying, 37,500 g, the coated granulated particles were continued with the drying process. The machine was turned off, and the coated granules were removed from the granulator. The coated granules were sized using a Granumill with a 7 mesh screen. The granulation was transferred to Tote Tumbler, mixed with 59 g of butylated hydroxytoluene and lubricated with 245 g magnesium stearate.

Next, the reboxetine drug composition and the push composition were compressed into tablets on a Manesty BB4 Tablet Press into a 5/16" (0.794 cm) diameter standard concave bilayered arrangement. First, 109 mg of the drug composition was added to the die cavity and pre-compressed using a 75 pounds force. Finally, 91 mg of the push composition is added and the layers are pressed under a pressure head of approximately 1000 pounds.

The bilayered arrangements were coated with a semi-permeable wall. The wall forming composition comprises 99% cellulose acetate having a 39.8% acetyl content and 1% polyethylene glycol comprising a 3350 viscosity-average molecular weight. The wall-forming composition is dissolved in an acetone:water (95:5 wt:wt) cosolvent to make a 5% solids solution. The wall-forming composition was sprayed onto and around the subcoated arrangements in a 24" Vector HiCoater.

Next, one 25 mil (0.0635 cm) exit passageway was drilled through the semi-permeable wall to connect the drug layer with the exterior of the dosage system. The residual solvent was removed by drying for 92 hours at 45° C and 45% humidity. Next, the osmotic systems were dried for 4 hours at 45° C to remove excess moisture.

The dosage form produced by this manufacture contains a drug layer composition of 4.80% reboxetine methanesulfonate, 83.68% POLYOX^{®} WSR N80 NF treated with phosphoric acid, 8.00% sodium chloride, 3.0% poly(vinylpyrrolidone) possessing a 40,000 molecular weight, 0.5% magnesium stearate, and 0.02% butylated hydroxytoluene.

The push composition comprises 64.2% poly(ethylene oxide) comprising a 7,000,000 molecular weight, 30% sodium chloride, 5% hydroxypropylmethylcellulose having an average molecular weight of 11,200, 0.5% ferric oxide, 0.05% butylated hydroxytoluene, and 0.25% stearic acid.

The semipermeable wall comprises 99 wt % cellulose acetate comprising a 39.8% acetyl content and 1% polyethylene glycol comprising a 3,350 viscosity-average molecular weight. The dosage form comprises one passageway, 25 mils (0.0635 cm), and it has a reboxetine base mean release rate of 0.3 mg/hr.

The dosage forms were packaged by placing 30 systems in each 45cc HDPE bottle along with a 1.5 g silica gel desciccant pack. The bottles were held at two conditions: 25°C and 60% RH as well as 40°C and 75% RH. Bottles were removed and samples tested at the timepoints shown in the table below.

| **Stability Time Point** | **N-Formyl Reboxetine (% of Reboxetine)** |
|---|---|
| Time = Zero | 0.10% |
| 1 Month at 40°C / 75% RH | 0.13% |
| 2 Months at 40°C / 75% RH | 0.20% |
| 1 Month at 25°C / 60% RH | 0.10% |
| 2 Months at 25°C / 60% RH | 0.10% |

### Example 11

### 4 mg, 5/16" standard round system made with methanesulfonic acid treated POLYOX^{®} N-80 with inorganic formate level of 55 ppm

Example 10 was repeated with the exception that the phosphoric acid treated POLYOX^{®} WSR N80 NF was replaced with methanesulfonic acid treated POLYOX^{®} WSR N80 NF. The inorganic formate level of this material is 55 PPM.

| **Stability Time Point** | **N-Formyl Reboxetine (% of Reboxetine)** |
|---|---|
| Time = Zero | 0.06% |
| 1 Month at 40°C / 75% RH | 0.11 % |
| 2 Months at 40°C / 75% RH | 0.06% |
| 3 Months at 40°C / 75% RH | 0.18% |
| 6 Months at 40°C / 75% RH | -- |
| 9 Months at 40°C / 75% RH | 0.42% |
| 12 Months at 40°C / 75% RH | 0.51 % |
| 1 Month at 25°C / 60% RH | 0.08% |
| 2 Months at 25°C / 60% RH | 0.03% |
| 3 Months at 25°C / 60% RH | 0.08% |
| 6 Months at 25°C / 60% RH | -- |
| 9 Months at 25°C / 60% RH | 0.13% |
| 12 Months at 25°C / 60% RH | 0.14% |

### Comparative Example 12

### 4 mg 9/32" standard round system made with untreated POLYOX^{®} N-80 with inorganic formate levels of greater than 500 ppm.

POLYOX^{®} WSR N80 NF with an inorganic formate level of over 500 PPM was used in the drug layer of an osmotic reboxetine delivery system that was manufactured as follows:

First, a binder solution was prepared. 350 g of poly(vinylpyrrolidone) identified as K29-32 having an average molecular weight of 40,000 was dissolved in 3150 g of water. 701.0 g of reboxetine methansulfonate, 8147 g of the untreated POLYOX^{®} WSR N80 NF, and 750 g of sodium chloride are added to a Freund Fluid Bed Granulator's bowl. The bowl was attached to the granulator and the granulation process was initiated for effecting granulation. Next, the dry powders were air suspended and mixed. Then, the binder solution was sprayed from a nozzle onto the powder. The granulating conditions were monitored during the process as follows: total solution spray rate of 120 g/min and inlet temperature of 40° C.

While spraying the binder solution, the filter bags were shaken for 10 seconds after every 30 second spray cycle to unglue any possible powder deposits. At the end of the solution spraying, 3500 g, the coated granulated particles were continued with the drying process. The machine was turned off, and the coated granules were removed from the granulator. The coated granules were passed through a 7 mesh screen using a Granumill. Next, the dried and screened granulation was transferred to a Gemco blender, mixed and lubricated 1.9 g of butylated hydroxytoluene for 10 minutes. Finally, 48.5 g of magnesium stearate was mixed into the granulation for 1 minute.

The push compostion was manufactured as in example B with the exception that poly(ethylene oxide) comprising a 2,000,000 molecular weight is substubstituted for poly(ethylene oxide) comprising a 7,000,000 molecular weight.

Next, the reboxetine drug composition and the push composition were compressed into tablets on a Manesty BB4 Tablet Press into a 9/32" (0.714 cm) diameter standard concave bilayered arrangement. First, 82 mg of the drug composition was added to the die cavity and pre-compressed using a 75 pounds force. Finally, 68 mg of the push composition is added and the layers were pressed under a pressure head of approximately 1000 pounds.

The bilayered arrangements were coated with a semi-permeable wall. The wall forming composition comprises 99% cellulose acetate having a 39.8% acetyl content and 1 % polyethylene glycol comprising a 3350 viscosity-average molecular weight. The wall-forming composition was dissolved in an acetone:water (95:5 wt:wt) cosolvent to make a 5% solids solution. The wall-forming composition was sprayed onto and around the subcoated arrangements in a 24" Vector HiCoater.

Next, one 30 mil (0.0762 cm) exit passageway was drilled through the semi-permeable wall to connect the drug layer with the exterior of the dosage system. The residual solvent was removed by drying for 92 hours at 45° C and 45% humidity. Next, the osmotic systems were dried for 4 hours at 45° C to remove excess moisture. The dosage form produced by this manufacture contains a drug layer composition of 7.01 % reboxetine methanesulfonate, 81.47% POLYOX^{®} WSR N80 NF, 7.50% sodium chloride, 3.5% poly(vinylpyrrolidone) possessing a 40,000 molecular weight, 0.5% magnesium stearate, and 0.02% butylated hydroxytoluene. The push composition comprises 63.7% poly(ethylene oxide) comprising a 2,000,000 molecular weight, 30% sodium chloride, 5% hydroxypropylmethylcellulose having an average molecular weight of 11,200, 1.0% ferric oxide, 0.05% butylated hydroxytoluene, and 0.25% magnesium stearate. The semipermeable wall comprises 99 wt % cellulose acetate comprising a 39.8% acetyl content and 1 % polyethylene glycol comprising a 3,350 viscosity-average molecular weight. The dosage form comprises one passageway, 30 mils (0.0762 cm), and it has a reboxetine base mean release rate of 0.3 mg/hr.

| **Stability Time Point** | **N-Formyl Reboxetine (% of Reboxetine)** |
|---|---|
| Time = Zero | 3.22% |
| 1 Month at 40°C / 75% RH | -- |
| 3 Months at 40°C / 75% RH | 3.88% |
| 6 Months at 40°C / 75% RH | 5.48% |
| 9 Months at 40°C / 75% RH | 3.97% |
| 1 Month at 25°C / 60% RH | -- |
| 3 Months at 25°C / 60% RH | 2.91 % |
| 6 Months at 25°C / 60% RH | 3.54% |

### Example 13

### 4mg, 3/16" LCT system made with methanesulfonic acid treated POLYOX N-80 with inorganic formate level of 85 ppm.

Example 10 was repeated with the exceptions that the phosphoric acid treated POLYOX^{®} WSR N80 NF was replaced with methanesulfonic acid treated POLYOX^{®} WSR N80 NF, with an inorganic formate level of 85 PPM. The bilayer tablets were compressed using a Carver Press. First, 145 mg of the reboxetine composition was added to the die cavity and pre-compressed, then, 97 mg of the push composition was added and the layers were pressed under a pressure head of approximately ½ a metric ton into a 3/16" (0.476 cm) diameter deep concave longitudinal layered arrangement.

| **Stability Time Point** | **N-Formyl Reboxetine (% of Reboxetine)** |
|---|---|
| Time = Zero | 0.04% |
| 1 Month at 40°C / 75% RH | -- |
| 3 Months at 40°C / 75% RH | 0.10% |
| 6 Months at 40°C / 75% RH | 0.13% |
| 1 Month at 25°C / 60% RH | -- |
| 3 Months at 25°C / 60% RH | 0.04% |
| 6 Months at 25°C / 60% RH | 0.08% |

## Claims

1. An osmotic dosage form comprising a reboxetine-containing component admixed with a reduced formate poly(alkylene oxide) polymer within an internal compartment, the internal compartment defined by a semipermeable membrane through which reboxetine is delivered via a delivery orifice formed or formable in the semipermeable membrane wherein said reduced formate polymer has a formic compound concentration of less than 200 ppmw.

2. An osmotic dosage form comprising a reboxetine-containing component and a reduced formate poly(alkylene oxide) polymer component configured as contacting adjacent layers within an internal compartment, the internal compartment defined by a semipermeable membrane through which reboxetine is delivered via a delivery orifice formed or formable in the semipermeable membrane at a location adjacent to the reboxetine-containing component, wherein said reduced formate polymer has a formic compound concentration of less than 200 ppmw.

3. The dosage form of Claim 1 or Claim 2 wherein the dosage form exhibits n-formyl formation of less than 0.76 % by weight of Reboxitine after 2 months at 40°C and 75% RH.

4. The dosage form of Claim 1 or Claim 2 wherein the dosage form exhibits n-formyl formation of less than 0.20 % by weight of Reboxitine after 2 months at 40°C and 75% RH.

5. The dosage form of Claim 1 or Claim 3 wherein the dosage form exhibits n-formyl formation of less than 0.51 % by weight of Reboxitine after 12 months at 40°C and 75% RH.

## Patentansprüche

1. Osmotische Dosierungsform umfassend eine Reboxetin enthaltende Komponente, die mit einem Poly(alkylenoxid)-Polymer von reduziertem Format innerhalb eines inneren Fachs vermischt ist, wobei das innere Fach definiert ist durch eine semipermeable Membran, durch die Reboxetin über eine Lieferöffnung geliefert wird, die in der semipermeablen Membran gebildet oder bildbar ist, wobei das Polymer von reduziertem Format eine Konzentration an Ameisensäureverbindung (formic compound) von weniger als 200 ppmw aufweist.

2. Osmotische Dosierungsform umfassend eine Reboxetin enthaltende Komponente und eine Poly(alkylenoxid)-Polymerkomponente von reduziertem Format, konfiguriert als sich berührende benachbarte Schichten innerhalb eines inneren Fachs, wobei das innere Fach durch eine semipermeable Membran definiert ist, durch die Reboxetin über eine Lieferöffnung geliefert wird, die in der semipermeablen Membran an einer Stelle angrenzend an die Reboxetin enthaltende Komponente gebildet oder bildbar ist, wobei das Polymer von reduziertem Format eine Konzentration an Ameisensäureverbindung von weniger als 200 ppmw aufweist.

3. Dosierungsform nach Anspruch 1 oder Anspruch 2, wobei die Dosierungsform eine n-Formylbildung von weniger als 0,76 Gew.-% an Reboxetin nach 2 Monaten, bei 40°C und 75 % RF, zeigt.

4. Dosierungsform nach Anspruch 1 oder Anspruch 2, wobei die Dosierungsform n-Formylbildung von weniger als 0,20 Gew.-% an Reboxetin nach 2 Monaten, bei 40°C und 75 % RF, zeigt.

5. Dosierungsform nach Anspruch 1 oder Anspruch 2, wobei die Dosierungsform n-Formylbildung von weniger als 0,51 Gew.-% an Reboxetin nach 12 Monaten, bei 40°C und 75 % RF, zeigt.

## Revendications

1. Forme de dosage osmotique comprenant un composant contenant de la réboxétine en mélange avec un polymère de formiate poly(oxyde d'alkylène) réduit dans un compartiment interne, le compartiment interne défini par une membrane semi-perméable à travers laquelle la riboxétine est délivrée via un orifice de délivrance formé ou formable dans la membrane semi-perméable, où ledit polymère de formiate réduit a une concentration en composé formique de moins de 200 ppm.

2. Forme de dosage osmotique comprenant un composant contenant de la riboxétine et un composant de polymère de formiate poly(oxyde d'alkylène) réduit configuré comme couches adjacentes se contactant dans un compartiment interne, le compartiment interne défini par une membrane semi-perméable à travers laquelle est délivrée la riboxétine via un orifice de délivrance formé ou formable dans la membrane semi-perméable en un emplacement adjacent au composant contenant la riboxétine, où ledit polymère de formiate réduit a une concentration en composé formique de moins de 200 ppm.

3. Forme de dosage de la revendication 1 ou la revendication 2, où la forme de dosage présente une formation de n-formyle de moins de 0,76% en poids de riboxétine après 2 mois à 40°C et 75% HR.

4. Forme de dosage de la revendication 1 ou la revendication 2, où la forme de dosage présente une formation de n-formyle de moins de 0,20% en poids de riboxétine après 2 mois à 40°C et 75% HR.

5. Forme de dosage de la revendication 1 ou la revendication 2, où la forme de dosage présente une formation de n-formyle de moins de 0,51% en poids de riboxétine après 2 mois à 40°C et 75% HR.
